(19) Europäisches Patentamt
European Patent Office
Office européen des brevets

(11) Publication number: **0 539 848 A1**

(12) # EUROPEAN PATENT APPLICATION

(21) Application number: **92117955.2**

(22) Date of filing: **21.10.92**

(51) Int. Cl.[5]: **C07C 323/51**, C07C 327/32, A61K 31/195, A61K 31/265

(30) Priority: **25.10.91 FR 9113174**

(43) Date of publication of application:
**05.05.93 Bulletin 93/18**

(84) Designated Contracting States:
**PT**

(71) Applicant: **INSTITUT NATIONAL DE LA SANTE ET DE LA RECHERCHE MEDICALE (INSERM)**
**101, rue de Tolbiac**
**F-75654 Paris Cédex 13(FR)**
Applicant: **RHONE-POULENC RORER SA**
**20, avenue Raymond Aron**
**F-92160 Antony(FR)**

(72) Inventor: **Fournie-Zaluski, Marie-Claude**
**16 avenue de Bouvines**
**F-75020 Paris(FR)**
Inventor: **Roques, Bernard-Pierre**
**12 rue Eugène Delacroix**
**F-94410 Saint Maurice(FR)**

(74) Representative: **Pilard, Jacques et al**
**RHONE-POULENC RORER S.A. 20 Avenue Raymond Aron**
**F-92165 Antony Cédex (FR)**

(54) **N-(mercaptoacyl)amino acids, methods of their preparation and therapeutic use, and pharmaceutical compositions containing them.**

(57) The present invention is directed to a compound of the formula (I)

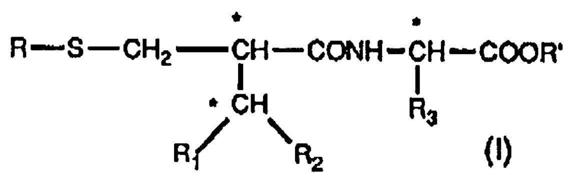

(I)

wherein

R represents a hydrogen atom or an acyl, aroyl or cycloalkylcarbonyl radical or a residue of formula

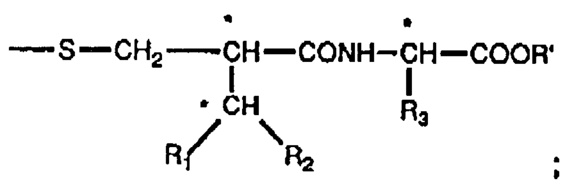

;

$R_1$ represents an alkyl radical;

$R_2$ represents an aryl or heteroaryl radical, or $R_1$ represents an alkylene chain attached to a carbon atom in an ortho position of the $R_2$ aryl or heteroaryl radical relative to a carbon of the $R_2$ aryl or heteroaryl radical linked to the propanoyl moiety;

$R_3$ represents a hydrogen atom or an alkyl, aryl, alkoxy or aryloxy radical; and

EP 0 539 848 A1

R' represents a hydrogen atom or an alkyl, aralkyl, acyl or aroyl radical;
and the pharmaceutically acceptable salts thereof.

The invention is also directed to the preparation of these compounds, pharmaceutical compositions comprising the compounds and methods for their pharmaceutical use.

Field of the Invention

The present invention is directed to compounds useful as extremely potent antihypertensives and that function by the concurrent inhibition of neutral endopeptidase and peptidyldipeptidase A. The invention is also directed to the preparation of these compounds, pharmaceutical compositions and methods for their pharmaceutical use.

Recent Developments

European Patent EP 0,038,758 describes products which are inhibitors of neutral endopeptidase (NEP) (EC 3.4-24.11). NEP is alternatively referred to as "enkephalinase" since the enzyme degrades the enkephalins which are endogenous opiate peptides of morphine receptors. These inhibitors are consequently useful as analgesics. This reference does not disclose that NEP inhibitors possess antihypertensive activity exhibited by inhibitors of peptidyldipeptidase A (ACE) (EC 3.4-15.1).

In Nature, Vol. 288, 285-288 (1980), Roques et al. have shown that (R,S)-(2-mercaptomethyl-3-phenylpropionyl)glycine (thiorphan) has an inhibitory power at a nanomolar concentration and behaves as an analgesic in potentiating the action of the enkephalins. This reference does not disclose that thiorphan possesses antihypertensive activity for example exhibited by inhibitors of ACE.

Other inhibitors of enkephalinase, endowed with analgesic properties, are the subject, for example, of French Patent FR 83 20024 (2,556,721) and European Patent EP 0,136,883. These references do not disclose that the NEP inhibitors possess antihypertensive activity for example exhibited by inhibitors of ACE.

U.S. Patent No. 4,879,309 discloses that compounds of formulae $HS-CH_2-CH(R_2)-CONH-CH(R_1)-COOR_3$ and $HS-(CH_2)_m-CH(R_2)-CONH-CH(R_1)-CO-A-COOR_3$ are useful for augmenting natriuresis and diuresis which thereby aids in reducing blood pressure. This reference does not disclose that the inhibitors possess antihypertensive activity separate from their natriuretic and diuretic effects.

Koehn et al., J. Biol. Chem., 262, 11623-11627 (1987) and S. L. Stephenson and A. J. Renny, Biochem. J., 243, 183-187 (1987) have reported that auricular natriuretic peptide which is liberated by the heart, particularly in cardiac insufficiency, and which augments natriuretic, diuretic and vasodilator effects, is inactivated by the peripheral enzyme EP 24.11. Thus, the inhibitor of NEP, thiorphan, and certain of its derivatives are capable of augmenting the half-life of circulating auricular natriuretic peptide and thereby aids in reducing blood pressure in the rat. [G. Olins et al, Moll. Cell. Endocrinol., 61, 201-208 (1989); A.A. Seymour et al., Hypertension, 14, 87-97 (1989)]. However, none of these references disclose that the NEP inhibitors possess antihypertensive properties exhibited by inhibitors of ACE activity.

Furthermore, in clinical trials, the inhibitors of neutral endopeptidase, such as thiorphan, produce natriuresis and diuresis without any significant hypotensive effect, except in certain sick people showing cardiac or renal insufficiency.

U. S. Patent Nos. 4,053,651 and 4,684,660 disclose inhibitors of peptidyldipeptidase A (ACE) (EC 3.4-15.1) which are useful as antihypertensives. These reference do not disclose that the ACE inhibitors have NEP inhibitory activity.

B.P. Roques et al., Trends in Pharmacological Sciences, Vol. 11 (6) 1989, have suggested combining in one molecule the properties of inhibition (1) of NEP, in order to potentiate the natriuretic, diuretic and vasorelaxant effects of atrial natriuretic peptide, and (2) of ACE, to block the hypertensive effects induced by anglotensin II formation. The reference does not disclose molecules capable of acting with both of these two enzymes at low concentrations.

The object of the present invention is directed to a compound that is an extremely potent antihypertensive that inhibits simultaneously NEP and ACE inhibitory properties at very low concentrations.

SUMMARY OF THE INVENTION

The present invention is directed to a compound of the formula (I)

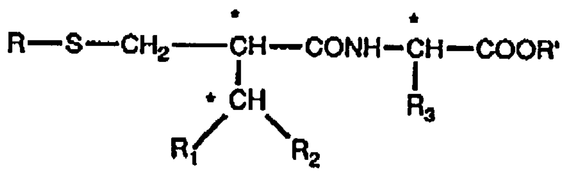

(I)

wherein

R represents a hydrogen atom or an acyl, aroyl or cycloalkylcarbonyl radical or a residue of formula (Ia)

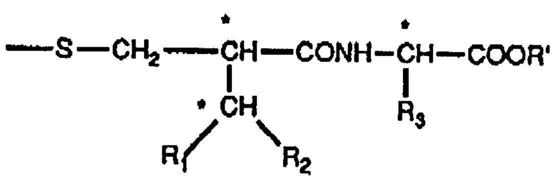

(Ia)

$R_1$ represents an alkyl radical;

$R_2$ represents an aryl or heteroaryl radical, or $R_1$ represents an alkylene chain attached to a carbon atom in on ortho position of the $R_2$ aryl or heteroaryl radical relative to a carbon of the $R_2$ aryl or heteroaryl radical linked to the propanoyl moiety;

$R_3$ represents a hydrogen atom or an alkyl, aryl, alkoxy or aryloxy radical; and

R' represents a hydrogen atom or an alkyl, aralkyl, acyl or aroyl radical;

and the pharmaceutically acceptable salts thereof.

DETAILED DESCRIPTION OF THE INVENTION

Definitions

As used above, and throughout the description of the invention, the following terms, unless otherwise indicated, shall be understood to have the following meanings:

"Patient" embraces both human beings and other mammals.

The "*" designation on the carbons in the compound according to the invention represents that the carbons are chiral.

"Alkyl" means an aliphatic hydrocarbon group which may be straight or branched having about 1 to about 17 carbon atoms in the chain, and the alkyl may be optionally substituted with one or more alkyl group substituents which may be the same or different, where "alkyl group substituent" includes halo, aryl, hydroxy, alkoxy, aryloxy, alkyloxy, alkylthio, arylthio, aralkyloxy, aralkylthio and cycloalkyl. Branched means that a lower alkyl group such as methyl, ethyl or propyl is attached to a linear alkyl chain. Preferred alkyl groups include the "lower alkyl" groups which are those alkyl groups having from about 1 to about 8 carbons. Exemplary alkyl groups are methyl, ethyl, *i*-propyl, t-butyl, heptyl, decyl or cyclohexylmethyl.

"Cycloalkyl" means a non-aromatic ring composed of about 4 to about 10 carbon atoms, and the cyclic alkyl may be partially unsaturated. Preferred cyclic alkyl rings include cyclopentyl, cyclohexyl. cycloheptyl, adamantyl, octahydronaphthyl and perhydronaphthyl. The cycloalkyl may be optionally substituted with an aryl group substituent.

"Alkenyl" means an alkyl group containing a carbon-carbon double bond. Exemplary groups include allyl and vinyl.

"Alkynyl" means an alkyl group containing a carbon-carbon triple bond. Exemplary groups include ethynyl and propargyl.

"Aryl" means aromatic carbocyclic radical containing about 6 to about 10 carbon atoms. Exemplary aryl include phenyl or naphthyl or phenyl or naphthyl substituted with one or more aryl group substituents which may be the same or different, where "aryl group substituent" includes alkyl, alkenyl, alkynyl, aryl, aralkyl, hydroxy, alkoxy, aryloxy, aralkoxy, carboxy, aroyl, halo, nitro, trihalomethyl, cyano, alkoxycarbonyl, aryloxycarbonyl, aralkoxycarbonyl, acylamino, aroylamino, carbamoyl, alkylcarbamoyl, dialkylcarbamoyl, arylthio, alkythio, alkylene or -NZZ' where Z and Z' are independently hydrogen, alkyl, aryl, or aralkyl.

"Heterocyclyl" means about a 5− to about a 10− membered monocyclic or multicyclic ring system in which one or more of the atoms in the ring or rings is an element other than carbon, for example nitrogen, oxygen or sulfur. "Substituted heterocyclyl" means a heterocyclyl group substituted by one or more aryl group substituents. Preferred heterocyclyl groups include furanyl, thienyl, pyridyl, pyrrolyl, N−methylpyrrolyl, quinolinyl, and isoquinolinyl.

"Acyl" means an alkyl−CO− group in which the alkyl group is as previously described. Preferred acyl have an alkyl containing about 1 to about 3 carbon atoms in the alkyl group. Exemplary groups include acetyl, propanoyl, 2−methylpropanoyl, butanoyl or palmitoyl.

"Aroyl" means an aryl−CO− group in which the alkyl group is as previously described. Exemplary groups include benzoyl and 1− and 2−naphthoyl.

"Alkoxy" means an alkyl−O− group in which the alkyl group is as previously described. Exemplary alkoxy groups include methoxy, ethoxy, *n*−propoxy, *i*−propoxy, *n*−butoxy and heptoxy.

"Aryloxy" means an aryl−O− group in which the aryl group is as previously described. Exemplary aryloxy groups include phenoxy and naphthoxy.

"Alkylthio" means an alkyl−S− group in which the alkyl group is as previously described. Exemplary alkylthio groups include methylthio, ethylthio, *i*−propylthio and heptylthio.

"Arylthio" means an aryl−S− group in which the aryl group is as previously described. Exemplary arylthio groups include phenthio and naphththio.

"Aralkyloxy" means an aralkyl−O− group in which the aralkyl group is a previously described. An exemplary aralkyloxy group is benzyloxy.

"Aralkylthio" means an aralkyl−S− group in which the aralkyl group is as previously described. An exemplary aralkylthio group is benzylthio.

"Dialkylamino" means an −NZZ' group wherein both Z and Z' are alkyl groups as previously described. Exemplary alkylamino groups include ethylmethylamino, dimethylamino and diethylamino.

"Alkoxycarbonyl" means an alkyl−O−CO− group. Exemplary alkoxycarbonyl groups include methoxy− and ethoxy− carbonyl.

"Aryloxycarbonyl" means an aryl−O−CO− group. Exemplary aryloxycarbonyl groups include phenoxy− and naphthoxy− carbonyl.

"Aralkoxycarbonyl" means an aralkyl−O−CO− group. An exemplary aralkoxycarbonyl group is benzyloxycarbonyl.

"Carbamoyl" is an $H_2N-CO-$ group.

"Alkylcarbamoyl" is an Z'ZN−CO− group wherein one of Z and Z' is hydrogen and the other of Z and Z' is alkyl as defined previously.

"Dialkylcarbamoyl" is an Z'ZN−CO− group wherein both Z and Z' are alkyl as defined previously.

"Acylamino" is an acyl−NH− group wherein acyl is as defined previously.

"Aroylamino" is an aroyl−NH− group wherein aroyl is as defined previously.

"Alkylene" means a straight or branched bivalent hydrocarbon chain group having from about 2 to about 8 carbon atoms, and the alkylene group may be interrupted by one or more substituted nitrogen atoms wherein the substituent is alkyl or oxygen or sulphur atoms, and it is presently more preferred that the alkylene group has from about 2 to about 3 carbon atoms. Exemplary alkylene groups include ethylene $(-CH_2CH_2-)$, propylene $(-CH_2-)_3$, $-CH_2-NMe-CH_2-$, $-O-CH_2-O-$ or $-O-(-CH_2-)_2-O-$.

"Halo" mean fluoro, chloro or bromo.

Description of the Preferred Embodiment

Presently preferred compounds are described by the formula (I) above wherein

R represents a hydrogen atom or an unbranched− or branched−chain acyl radical containing 1 to 18 carbon atoms, an aroyl radical containing 6 to 10 carbon atoms, a cycloalkylcarbonyl radical In which the cycloalkyl portion contains 4 to 10 carbon atoms or a residue of formula (Ia) as described above;

$R_1$ represents an optionally substituted, unbranched− or branched−chain alkyl radical containing 1 to 8 carbon atoms;

$R_2$ represents an aryl or heteroaryl radical containing 5 to 10 carbon atoms, optionally substituted at a position other than the ortho position with respect to the carbon linked to the propanoyl chain and in which the carbon atom at the ortho position with respect to the carbon linked to the propanoyl chain optionally forms with $R_1$ an unbranched or branched alkylene chain containing 2 to 8 carbon atoms, optionally interrupted by one or more substituted nitrogen atoms or oxygen or sulphur atoms;

$R_3$ represents a hydrogen atom or an optionally substituted, unbranched, or branched−chain alkyl

radical containing 1 to 8 carbon atoms, an optionally substituted aryl or heteroaryl radical containing 5 to 10 carbon atoms, an optionally substituted alkoxy radical containing 1 to 8 carbon atoms or an optionally substituted aryloxy radical containing 6 to 10 carbon atoms; and

R' represents a hydrogen atom or an unbranched- or branched-chain alkyl radical containing 1 to 8 carbon atoms, an aralkyl radical in which the aryl portion contains 6 to 10 carbon atoms and in which the unbranched- or branched-chain alkyl portion contains 1 to 8 carbon atoms, an acyl radical containing 2 to 18 carbon atoms or an aroyl radical in which the aryl portion contains 6 to 10 carbon atoms and is optionally substituted.

More preferred are compounds of formula (I) above wherein

R represents a hydrogen atom or an acyl radical containing 2 to 4 carbon atoms, a benzoyl radical in which the phenyl ring is optionally substituted with a halogen (chlorine, bromine, fluorine) atom or with a hydroxyl radical, an alkoxy radical containing 1 to 4 carbon atoms, an amino radical, a dialkylamino radical in which each alkyl portion contains 1 to 4 carbon atoms or a trifluoromethyl radical, an adamantoyl, palmitoyl or pamoyl radical or a residue of formula (Ia) above;

$R_1$ represents a methyl or trifluoromethyl radical,

$R_2$ represents:

- either a phenyl, 2-, 3- or 4-pyridyl, N-methyl-2- or -3-pyrrolyl, 2-or 3-furyl or 2- or 3-thienyl radical optionally substituted, at a position other than the ortho position with respect to the carbon linked to the propanoyl chain, with one or more identical or different atoms or radicals chosen from halogen (chlorine, bromine, fluorine) atoms and hydroxyl radicals, unbranched- or branched-chain acyloxy radicals containing 1 to 4 carbon atoms, unbranched-or branched-chain alkoxy radicals containing 1 to 4 carbon atoms, phenoxy, phenylthio and amino radicals, dialkylamino radicals in which each alkyl portion contains 1 to 4 carbon atoms and methylenedioxy or ethylenedioxy radicals;
- or a phenyl, 2-, 3- or 4-pyridyl, N-methyl-2- or -3-pyrrolyl, 2- or 3-furyl or 2- or 3-thienyl radical in which the carbon atom at the ortho position with respect to the carbon atom linked to the propanoyl chain forms with $R_1$ an alkylene radical containing 2 or 3 carbon atoms optionally substituted with a methyl radical or a radical $-CH_2-Y$ or $-Y-CH_2-$ in which Y represents an oxygen or sulphur atom or a nitrogen atom substituted with a methyl radical;

$R_3$ represents a hydrogen atom or a trifluoromethyl radical or

- an unbranched- or branched-chain alkyl radical containing 1 to 8 carbon atoms, optionally substituted with a phenyl radical or with a hydroxyl radical or with an alkoxy radical containing 1 to 4 carbon atoms or with a phenoxy radical or with an alkylthio radical containing 1 to 4 carbon atoms or with a phenylthio radical or with a benzyloxy or benzylthio radical,
- an alkoxy radical containing 1 to 8 carbon atoms, or
- a phenoxy radical, or
- an aryl radical such as a phenyl or thienyl radical,

with the understanding that the phenyl or aryl radicals and the phenyl portions of the phenoxy, phenylthio, benzyloxy or benzylthio radicals are optionally substituted with one or more identical or different atoms or radicals chosen from halogen (chlorine, bromine, fluorine) atoms and hydroxyl radicals, alkoxy radicals containing 1 to 4 carbon atoms, amino radicals, dialkylamino radicals in which each alkyl portion contains 1 to 4 carbon atoms and methylenedioxy or ethylenedioxy radicals; and

R' represents a hydrogen atom or a methyl, ethyl, benzyl, cyclohexylmethyl, palmitoyl or pamoyl radical.

The compounds of the present invention may be useful in the form of the free base or acid or in the form of a pharmaceutically acceptable salt thereof. All forms are within the scope of the invention.

Where the compound of the invention is substituted with an acidic moiety, base addition salts may be formed and are simply a more convenient form for use; in practice, use of the salt form inherently amounts to use of the free acid form. The bases which can be used to prepare the base addition salts preferably include those which produce, when combined with the free acid, pharmaceutically acceptable salts, that is, salts whose cations are non-toxic to the animal organism in pharmaceutical doses of the salts, so that the beneficial mixed inhibition of NEP and ACE inherent in the compound of the invention is not vitiated by side effects ascribable to the cations. Although pharmaceutically acceptable salts of said acidic compounds are preferred, all base addition salts are useful as sources of the free acid form even if the particular salt, per se, is desired only as an intermediate product as for example, when the salt is formed only for purposes of purification, and identification, or when it is used as intermediate in preparing a pharmaceutically acceptable salt by ion exchange procedures. Pharmaceutically acceptable salts within the scope of the invention

include those derived from the following bases: sodium hydroxide, potassium hydroxide, calcium hydroxide, aluminum hydroxide, lithium hydroxide, magnesium hydroxide, zinc hydroxide, ammonia, ethylenediamine, N-methylglucamine, lysine, arginine, ornithine, choline, N,N'-dibenzylethylenediamine, chloroprocaine, diethanolamine, procaine, N-benzylphenethylamine, diethylamine, piperazine, tris(hydroxymethyl)-aminomethane, tetramethylammonium hydroxide, and the like.

Metal salts of compounds of the present invention may be obtained by contacting a hydroxide, carbonate or similar reactive compound of the chosen metal in an aqueous solvent with the free acid form of the compound. The aqueous solvent employed may be water or it may be a mixture of water with an organic solvent, preferably an alcohol such as methanol or ethanol, a ketone such as acetone, an aliphatic ether such as tetrahydrofuran, or an ester such as ethyl acetate. Such reactions are normally conducted at ambient temperature but they may, if desired, be conducted with heating.

Amino salts of compounds of the present invention may be obtained by contacting an amine in an aqueous solvent with the free acid form of the compound. Suitable aqueous solvents include water and mixtures of water with alcohols such as methanol or ethanol, ethers such as tetrahydrofuran, nitriles such as acetonitrile, or ketones such as acetone. Amino acid salts may be similarly prepared.

Preferred base addition salts have a cation selected from the group consisting of ammonium, sodium, calcium, protonated N-methyl-D-glucamine, protonated lysine, protonated arginine and protonated dicyclohexylamine.

Where the compound of the present invention is substituted with a basic moiety, add addition salts may be formed and are simply a more convenient form for use; and in practice, use of the salt form inherently amounts to use of the free base form. The acids which can be used to prepare the acid addition salts include preferably those which produce, when combined with the free base, pharmaceutically acceptable salts, that is, salts whose anions are non-toxic to the animal organism in pharmaceutical doses of the salts, so that the beneficial mixed inhibition of NEP and ACE inherent in the compound of the invention is not vitiated by side effects ascribable to the anions. Pharmaceutically acceptable salts within the scope of the invention are those derived from the following acids: mineral acids such as hydrochloric acid, sulfuric acid, phosphoric acid and sulfamic acid; and organic acids such as acetic acid, citric acid, lactic acid, tartaric add, malonic acid, methanesulfonic acid, ethanesulfonic acid, benzenesulfonic acid, *p*-toluenesulfonic acid, cyclohexylsulfamic acid, quinic acid, and the like. The corresponding acid addition salts comprise the following: hydrochloride, sulfate, phosphate, sulfamate, acetate, citrate, lactate, tartarate, malonate, methanesulfonate, ethanesulfonate, benzenesulfonate, *p*-toluenesulfonate, cyclohexylsulfamate and quinate, respectively.

The acid addition salts of the compounds of this invention are prepared either by dissolving the free base in aqueous or aqueous-alcohol solution or other suitable solvents containing the appropriate acid and isolating the salt by evaporating the solution, or by reacting the free base and acid in an organic solvent, in which case the salt separates directly or can be obtained by concentration of the solution.

The compound according to the invention contains several asymmetric centers and may exist in the form of pure stereoisomers or in the form of mixtures of stereoisomers. The carbon atoms marked by an asterisk (*) designate asymmetric centers. In the compound according to the invention, unless stated otherwise, designations regarding the stereochemical configurations at the carbon atoms marked by an asterisk relate: first to the carbon atom bearing the substituents $R_1$ and $R_2$; second to central carbon atom of the propanoyl chain; and third to the carbon atom bearing the substituent $R_3$. Preferred compounds according to the invention include those wherein any of the carbon atoms marked by an asterisk (*) have the S configuration. Preferred also include the compounds according to the invention having a stereochemical designation selected from the group consisting of R,R,S, S,R,S, S,S,S and R,S,S form, and it is more preferred having a stereochemicai designation S,S,S or R,S,S.

According to the invention, the N-(mercaptoacyl)amino acids of general formula (I) may be prepared by the acylation of an amino acid of formula (II)

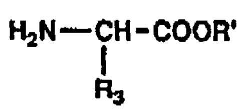

(II)

in which $R_3$ and R' are defined as above, by means of an acid of formula (III)

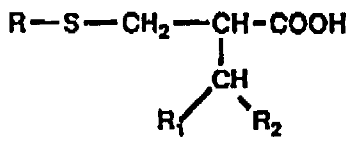

(III)

in which R, $R_1$ and $R_2$ are defined as above, under the customary conditions used in peptide chemistry, described, for example, by Bodansky et al., "Peptide Synthesis", J. Wiley and Sons Edit.

More especially, the acylation is performed in the presence of a condensing agent such as dicyclohexylcarbodiimide, optionally in the presence of 1-hydroxybenzotriazole.

The products of general formula (III) may be obtained by the Michael addition of a sulphur derivative of formula (IV)

R-SH (IV)

in which R is defined as above, to an acrylic acid of formula (V)

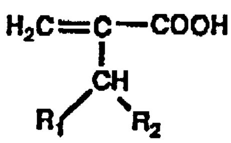

(V)

in which $R_1$ and $R_2$ are defined as above,

Preferably, thioacetic acid or thiobenzoic acid is used as a sulphur derivative of formula (IV).

The acrylic acid of formula (V) may be obtained by saponification of a corresponding ester in a basic medium. Preferably, the methyl or ethyl ester is used.

The acrylic ester of formula (VI)

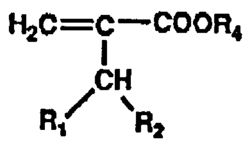

(VI)

in which $R_1$ and $R_2$ are defined as above and $R_4$ preferably represents an alkyl radical containing 1 to 4 carbon atoms, may be obtained by a Mannich reaction on the monoester of formula (VII)

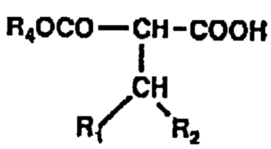

(VII)

in which $R_1$, $R_2$ and $R_4$ are defined as above.

Generally, the Mannich reaction is performed by means of formaldehyde in the presence of a secondary amine such as diethylamine at a temperature of about 20 °C.

The monoester of formula (VII) is obtained by monosaponification in a basic medium of a malonic ester of formula

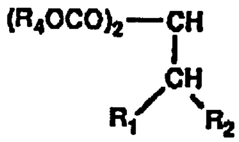

(VIII)

in which $R_1$, $R_2$ and $R_4$ are defined as above,

The malonic ester of formula (VIII) may be obtained by the action of a halide of formula (IX)

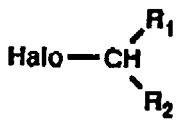

(IX)

in which $R_1$ and $R_2$ are defined as above and Halo represents a halide atom, preferably a bromide atom, on an alkyl malonate anionised beforehand, for example by means of an alkali metal alcoholate optionally prepared in situ. The malonic ester of formula (VIII) may also be obtained by condensation of a ketone of formula (X)

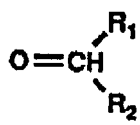

(X)

in which $R_1$ and $R_2$ are defined as above, with an alkyl malonate anionised beforehand as described above, to form the product of formula (XI)

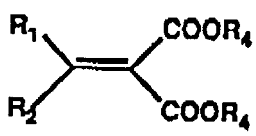

(XI)

in which $R_1$, $R_2$ and $R_4$ are defined as above, which is reduced catalytically, for example with hydrogen in the presence of palladium on charcoal, to the product of formula (VIII).

The product of formula (VI) may also be obtained by a Wittig reaction with formaldehyde on a phosphonate of formula (XII)

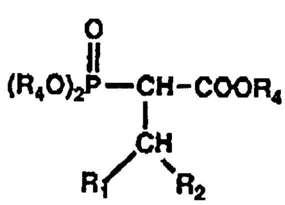

(XII)

in which $R_1$, $R_2$ and $R_4$ are defined as above.

The product of formula (XII) may be obtained by the action of a product of formula (IX) on an alkyl phosphonoacetate of formula (XIII)

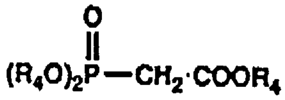

(XIII)

in which $R_4$ is defined as above.

The reaction is generally performed in the presence of a strong base such as sodium hydride.

The products of general formula (I) in which R and R' each represent a hydrogen atom may be obtained from a product of general formula (I) in which R and R' are other than a hydrogen atom. More especially, when R represents an acyl radical and R' represents an alkyl or phenylalkyl radical, their replacement by a hydrogen atom is generally performed by hydrolysis in an alkaline medium, working in an inert medium so as to avoid oxidation of the mercapto group.

The resolved forms of the compounds according to the invention may be prepared by standard practices known to those skilled in the art such as fractional crystallization and column chromatography. For example, compounds of formula (III) such as an acylthioalkanoic acid may be resolved by fractional recrystallization with optically active bases such as methylbenzylamine or 1-(1-naphthyl)ethylamine and then the resolved compounds of formula (III) may be reacted with an optically active amino acid according to the acylation reaction described above to yield compounds of formula (I).

The present invention is further exemplified but not limited by the following illustrative examples.

I - PREPARATION OF THE INTERMEDIATES

EXAMPLE A

2-Acetylthiomethyl-3-phenylbutanoic Acid

Ethyl malonate (4.1 mL) is added to a solution of sodium ethoxide prepared from sodium metal (0.75 g) and ethanol (40 mL). 1-Bromo-1-phenylethane (6 g) is added at 0 ° C and the mixture is then stirred for 20 hours at 30 ° C. After concentrating to dryness, the residue is taken up with water (100 mL) and then extracted three times with ethyl acetate (75 mL). The organic phase is washed with water and then with saturated sodium chloride solution and finally dried over magnesium sulphate. After filtering and concentrating to dryness, ethyl (1-phenylethyl)malonate (6 g) is obtained in an 84 % yield in the form of a pale yellow oil, having the following characteristics: - $R_f$ = 0.67 [hexane/ethyl acetate (65:35 by volume)].

The ethyl (1-phenylethyl)malonate is stirred overnight with sodium hydroxide (1.3 eq) in an acetone/water (3:1 by volume) mixture. After concentrating to dryness, the residue is taken up with water (40 mL) and the mixture is then extracted three times with ethyl acetate (25 mL). The organic phase is washed, dried, filtered and then concentrated to dryness to yield 3.5 g of 2-ethoxycarbonyl-3-phenylbutanoic acid in a 65 % yield in the form of an oil, having the following characteristics: - $R_f$ = 0.50 [methylene chloride/methanol (9:1 by volume)].

To the 2-ethoxycarbonyl-3-phenylbutanoic acid (3.5 g) is added diethylamine (1.53 mL, 1 eq) and 30 % formaldehyde (1.78 mL) at 0 ° C. The mixture is stirred overnight at a temperature of about 20 ° C and is then taken up with diethyl ether (100 mL). The organic phase is separated after settling takes place, washed with 10 % aqueous citric acid solution (40 mL), with water (40 mL) and with saturated sodium chloride solution (40 mL) and finally dried over sodium sulphate. After filtering and concentrating to dryness, 2.2 g of ethyl 2-phenylethyl)acrylate is obtained in a 71 % yield in the form of a yellow oil, having the following characteristics: - $R_f$ = 0.86 [methylene chloride/methanol (9:1 by volume)].

The product thereby obtained (2.2 g) is treated with of sodium hydroxide (1.5 eq) in an acetone/water (2:1 by volume) mixture overnight at a temperature of about 20 ° C. The mixture is concentrated to dryness and the residue is then taken up with water (30 ml). The aqueous phase is acidified to pH 2 by adding hydrochloric acid.

The product is then extracted three times with ethyl acetate (20 mL). The organic phase is washed with water (15 mL) and then with saturated sodium chloride solution (15 mL) and finally dried over sodium sulphate. After filtering and concentrating to dryness, 1.8 g of 2-(1-phenylethyl)acrylic acid is obtained in a 95 % yield in the form of a white solid, having the following characteristics: - melting point (m.p.); 115 ° C - $R_f$ = 0.61 [methylene chloride/methanol (9:1 by volume)].

The acid thereby obtained (1.5 g) is heated to 80 ° C with thioacetic acid (6 mL). After concentration to dryness, 0.21 g of 2-acetylthiomethyl-3-phenylbutanoic acid is obtained in a 98 % yield in the form of a

yellow oil, having the following characteristics: – $R_f$ = 0.73 [methylene chloride/methanol (9:1 by volume)].

EXAMPLE B

2-Acetylthiomethyl-3-phenylbutanoic Acid

Sodium hydride (4.15 g) in 80 % suspension in oil is added to a solution of triethyl phosphonoacetate (25 mL) in anhydrous dimethylformamide (36 mL). After 15 minutes at 0°C, I-bromo-1-phenylethane (18 mL, 1.05 eq) is added. The mixture is stirred overnight at a temperature of about 20°C under a nitrogen atmosphere. After evaporation of dimethylformamide, the residue is taken up with ethyl acetate (200 mL). The organic phase is washed three times with water (60 mL) and then with saturated sodium chloride solution (60 mL) and finally dried over sodium sulphate. After filtering and concentrating to dryness, 40.3 g of diethyl 2-(1-phenylethyl)phosphonoacetate is obtained in a 97 % yield in the form of an oil, having the following characteristics: – $R_f$ = 0.57 [cyclohexane/ethyl acetate/acetic acid(5:5:0.5 by volume)].

The diethyl 2-(1-phenylethyl)phosphonoacetate (40.3 g) is dissolved in 37 % formaldehyde (65 mL), and potassium carbonate (51 g) is then added. The mixture is heated to reflux for 3 hours 30 minutes. After cooling, the mixture is taken up with hexane (400 mL). The organic phase is washed with water twice 100 mL) and then with saturated sodium chloride solution (100 mL) and finally dried over sodium sulphate. After filtering and concentrating to dryness, 24.2 g of ethyl 2-(1-phenylethyl)acrylate are obtained in a 96 % yield in the form of an oil, the characteristics of which are identical to those of the product obtained in Example A.

EXAMPLE C

3-Acetylthio-2-(1-indanyl)propanoic Acid

Using the procedure described in Example B, but starting with 1-bromoindane and triethyl phosphonoacetate, ethyl 2-(1-indanyl)acrylate is obtained, which product, by the action of thioacetic acid under the conditions described in Example A, yields 3-acetylthio-2-(1-indanyl)propanoic acid in the form of a pale yellow oily product, having the following characteristics: $R_f$ = 0.60 [hexane/ethyl acetate/acetic add (5:5:0.5 by volume)].

EXAMPLE D

2-Acetylthiomethyl-3-(4-hydroxyphenyl)butanoic Acid

Using the procedure described in Example A, but starting with 1-bromo-1-(4-hydroxyphenyl)-ethane, 2-acetylthiomethyl-3-(4-hydroxyphenyl)butanoic acid is obtained, having the following characteristics: – m.p.: 48°C – $R_f$ = 0.58 [methylene chloride/methanol (9:1 by volume)].

EXAMPLE E

2-Acetylthiomethyl-3-[(4-fluoro)phenyl]butanoic Acid

Working as in Example A, but starting with 1-bromo-1-[(4-fluoro)phenyl]ethane, 2-acetylthiomethyl-3-[(4-fluoro)phenyl]butanoic acid is obtained in a 98% yield as a thick oil having the following characteristics: – $R_f$ = 0.38 [n-hexane/ethyl acetate/acetic acid (7:3:0.5 by volume)].

EXAMPLE F

2-Acetylthiomethyl-3-[(3,4-difluoro)phenyl]butanoic Acid

Working as in Example A, but starting with 1-bromo-1-[(3,4-difluoro)phenyl]ethane, 2-acetylthiomethyl-3-[(3,4-difluoro)phenyl]-butanoic acid is obtained in a 95% yield as a thick oil having the following characteristics: $R_f$ = 0.33 [hexane/ethyl acetate (1:1 by volume)].

## 2 – PREPARATION OF THE PRODUCTS ACCORDING TO THE INVENTION

EXAMPLE 1 – N–(2–acetylthiomethyl–1–oxo–3–phenylbutyl)tyrosine Benzyl Ester

2–acetylthiomethyl–3–phenylbutanoic acid (5 g) are dissolved in dry tetrahydrofuran (25 mL). A solution of (S)–tyrosine benzyl ester p–tosylate (1 equivalent) and triethylamine (1 eq) in chloroform (25 mL), a solution of 1–hydroxybenzotriazole (1 eq) in tetrahydrofuran (30 mL) and a solution of dicyclohexylcarbodiimide (1.2 eq) in chloroform (25 mL) are added successively at 0°C. The mixture is stirred for 1 hour at 0°C and then overnight at a temperature in the region of 20°C. After filtering, the mixture is concentrated to dryness and the residue is then taken up with ethyl acetate (80 mL). The organic phase is washed successively with 10 % citric acid solution (20 mL), with water (20 mL), with saturated sodium bicarbonate solution (20 mL), with water (20 mL) and then with saturated sodium chloride solution (20 mL). After drying over sodium sulphate, filtering and concentrating to dryness, 8 g of N–(2–acetylthiomethyl–1–oxo–3–phenylbutyl)tyrosine benzyl ester is obtained in an 80% yield in the form of a white solid, having the following characteristics: – $R_f$ = 0.65 [chloroform/methanol (9.5:0.5 by volume)].

The product is purified by chromatography on silica.

EXAMPLE 2 – N–(2–mercaptomethyl–1–oxo–3–phenylbutyl)tyrosine

1 g of the product obtained in Example 1 is dissolved in an degassed acetone/water (2:1 by volume) mixture. 1M sodium hydroxide (4 eq) is added at 0°C and under a nitrogen atmosphere. The mixture is stirred for 5 hours at a temperature of about 20°C. After filtering and acidifying to pH 1, the product is extracted three times with degassed chloroform (15 mL). The organic phase is dried over sodium sulphate. After filtering and concentrating to dryness, 0.73 g of N–(2–mercaptomethyl–l–oxo–3–phenylbutyl)–tyrosine is obtained in an 81% yield, the characteristics of which product are as follows: – m.p.: 80°C – $R_f$ = 0.43 [methylene chloride/methanol/acetic acid (9:1:0.5 by volume)].

EXAMPLE 3 – N–(acetylthiomethyl–1–oxo–3–phenylbutyl)glycine Benzyl Ester

Using the procedure described in Example 1, starting with 2–acetylthiomethyl–3–phenylbutanoic acid and glycine benzyl ester, and after purifying by chromatography, eluting with a cyclohexane/ethyl acetate (6:4 by volume) mixture, N–(acetylthiomethyl–1–ox–3–phenylbutyl)glycine benzyl ester is obtained in a 61 % yield, the characteristics of which product are as follows: – $R_f$ = 0.39 [cyclohexane/ethyl acetate(6:4 by volume)].

EXAMPLE 4 – N–(2–mercaptomethyl–1–oxo–3–phenylbutyl)glycine

Using the procedure described in Example 2, starting with the product obtained in Example 3, N–(2–mercaptomethyl–1–oxo–3–phenylbutyl)glycine is obtained in an 89 % yield, the characteristics of which product are as follows: – m.p.: 129°C – $R_f$ = 0.62 [methylene chloride/methanol/acetic acid (9:1:0.5 by volume)].

EXAMPLE 5 – N–(2–acetylthiomethyl–1–oxo–3–phenylbutyl)–O–benzylserine Benzyl Ester

Using the procedure described in Example 1, starting with 2–acetylthiomethyl–3–phenylbutanoic acid and O–benzylserine benzyl ester, and after purification by chromatography, eluting with a hexane/ethyl acetate (8:2 by volume) mixture, N–(2–acetylthiomethyl–1–oxo–3–phenylbutyl)–O–benzylserine benzyl ester is obtained in a 62 % yield, the characteristics of which product are as follows: – $R_f$ = 0.16 [hexane/ethyl acetate (8:2 by volume)].

EXAMPLE 6 – N–(2–mercaptomethyl–1–oxo–3–phenylbutyl)–O–benzylserine

Using the procedure described in Example 2, starting with the product obtained in Example 5, N–(mercaptomethyl–l–oxo–3–phenylbutyl)–O–benzylserine is obtained in a 72 % yield in the form of an oil, having the following characteristics: – $R_f$ = 0.29 [methylene chloride/methanol (9:1 by volume)].

EXAMPLE 7 – N-(2-acetylthiomethyl-1-oxo-3-phenylbutyl)alanine Benzyl Ester

Using the procedure described in Example 1, starting with 2-acetylthiomethyl-3-phenylbutanoic acid and alanine benzyl ester, and after purification by chromatography, eluting with a cyclohexane/ethyl acetate (75:25 by volume) mixture, N-(2-acetylthiomethyl-1-oxo-3-phenylbutyl)alanine benzyl ester is obtained in a 68 % yield, the characteristics of which product are as follows:- $R_f$ = 0.31 [hexane/ethyl acetate (75:25 by volume)].

EXAMPLE 8 – N-(2-mercaptomethyl-1-oxo-3-phenylbutyl)alanine

Using the procedure described in Example 2, starting with the product obtained in Example 7, N-(2-mercaptomethyl-1-oxo-3-phenylbutyl)alanine is obtained in a 75% yield in the form of a colorless oil, having the following characteristics:- $R_f$ = 0.27 [methylene chloride/methanol (9:1 by volume)].

XAMPLE 9 – N-(2-acetylthiomethyl-1-oxo-3-phenylbutyl)norleucine Benzyl Ester

Using the procedure described in Example 1, but starting with 2-acetylthiomethyl-3-phenylbutanoic acid and norleucine benzyl ester, and after purifying by chromatography, eluting with a hexane/ethyl acetate (75:25 by volume) mixture, N-(2-acetylthiomethyl-1-oxo-3-phenylbulyl)norleucine benzyl ester is obtained in a 75 % yield in the form of an oil, having the following characteristics:- $R_f$ = 0.46 [hexane/ethyl acetate (6.5:3.5 by volume)],

EXAMPLE 10 – N-(2-mercaptomethyl-1-oxo-3-phenylbutyl)norleucine

Using the procedure described in Example 2, but starting with the product obtained in Example 9, N-(2-mercaptomethyl-1-oxo-phenylbutyl)norleucine is obtained in a 90 % yield, the characteristics of which product are as follows:- m.p.: 55 °C – $R_f$ = 0.47 [methylene chloride/methanol (9:1 by volume)].

EXAMPLE 11 – N-[3-acetylthio-2-(1-indanyl)-1-oxopropyl]alanine Benzyl Ester

Using the procedure described in Example 1, but starting with 3-acetylthio-2-(1-indanyl)propanoic acid and alanine benzyl ester, N-[3-acetylthio-2-(1-indanyl)-1-oxopropyl)alanine benzyl ester is obtained in a 72 %, yield in the form of a white solid, having the following characteristics: – m.p.: 95 °C – $R_f$ = 0.17 [cyclohexane/ethyl acetate (4:1 by volume)].

EXAMPLE 12 – N-[3-mercapto-2-(1-indanyl)-1-oxopropyl]alanine

Using the procedure described in Example 2, but starting with the product obtained in Example 11, N-[3-mercapto-2-(1-indanyl)-1-oxopropyl]-alanine is obtained in a 96 % yield, the characteristics of which product are as follows:- m.p.: 121 °C – $R_f$ = 0.22 [methylene chloride/methanol (9:1 by volume).

EXAMPLE 13 – N-[2-acetylthiomethyl-3-(4-hydroxyphenyl)-1-oxobutyl]alanine Benzyl Ester

Using the procedure described in Example 1, but starting with 2-acetylthiomethyl-3-(4-hydroxyphenyl)butanoic acid and alanine benzyl ester, N-[2-acetylthiomethyl-3-(4-hydroxyphenyl)-1-oxobutyl]alanine benzyl ester is obtained in a 69 % yield, the characteristics of which product are as follows: $R_f$ = 0.22 [hexane/ethyl acetate (65:35 by volume)].

EXAMPLE 14 – N-[2-mercaptomethyl-3-(4-hydroxyphenyl)-1-oxobutyl]alanine

Using the procedure described in Example 2, but starting with the product obtained in Example 13, N-[2-mercaptomethyl-3-(4-hydroxyphenyl)-1-oxobutyl]alanine is obtained in an 85 % yield in the form of a white solid, having the following characteristics:- m.p.: 72 °C – $R_f$ = 0.18 [methylene chloride/methanol (9:1 by volume)].

EXAMPLE 15 – N-[2-acetylthiomethyl-3-(4-hydroxyphenyl)-1-oxobutyl]tyrosine Benzyl Ester

Using the procedure described in Example 1, but starting with 2-acetylthiomethyl-3-(4-hydroxyphenyl)butanoic acid and tyrosine benzyl ester, N-[2-acetylthiomethyl-3-(4-hydroxyphenyl)-1-oxobutyl]tyrosine benzyl ester is obtained in a 78 % yield in the form of a white solid, having the following characteristics: – $R_f$ = 0.16 [hexane/ethyl acetate (65:35 by volume)].

EXAMPLE 16 – N-[2-mercaptomethyl-3-(4-hydroxyphenyl)-1-oxobutyl]tyrosine

Using the procedure described in Example 2, but starting with the product obtained in Example 15, N-[2-mercaptomethyl-3-(4-hydroxyphenyl)-1-oxobutyl]tyrosine is obtained in a 70% yield in the form of a white solid, having the following characteristics:– m.p.: 115 °C – $R_f$ = 0.27 [methylene chloride/methanol (8:2 by volume)].

EXAMPLE 17 – N-[2-adamantoylthiomethyl-1-oxo-3-phenylbutyl]alanine

The product (1.7 g) of Example 8 is dissolved in degassed water (18 mL). To the solution under a nitrogen atmosphere and at 0 °C is added a solution 1.3 X $10^{-2}$ M sodium hydroxide (2.2 eq) and adamantoyl chloride (1.33 g, 1.1 eq). The mixture is stirred for four hours at ambient temperature. The mixture is then acidified to pH 3 with 1M hydrochloric acid and extracted with ethyl acetate. The organic phase is washed with water and then a saturated NaCl solution. The organic phase is dried over anhydrous $Na_2SO_4$, filtered and evaporated to dryness. A white solid (2 g) is obtained in a yield of 74% having the following characteristics: m.p 80 °C; $R_f$ = 0.45 [chloroform/methanol/ acetic acid (7:3:0.3 by volume)].

EXAMPLE 18 – N-[2-acetylthiomethy-3-(4-fluorophenyl)-1-oxobutyl]alanine Benzyl Ester

Working as in Example 1, but starting with 2-acetylthiomethyl-3-(4-fluorophenyl)butanoic acid and benzyl esters of alanine, the titled compound is obtained in a yield of 73% as an oil having the characteristics: $R_f$ = 0.23 [cyclohexane/ethyl acetate (7:3 by volume)].

EXAMPLE 19 – N-[2-acetylthiomethyl-3-(4-fluorophenyl)-1-oxobutyl]alanine

Working as in Example 2, but starting with the product of Example 18, the titled compound is obtained in a yield of 78% as a white solid having the following characteristics: m.p. 93 °C; $R_f$ = 0.5 [methylene chloride/methanol/acetic acid (9:1:0.5 by volume)]

EXAMPLE 20 – N-[2-acetylthio-3-(4-fluorophenyl)-1-oxobutyl]tyrosine Benzyl Ester

Working as in Example 1, but starting with 2-acetylthio-3-(4-fluorophenyl)butanoic acid and benzyl ester of lysine, the titled compound is obtained in a yield of 89% as a pale yellow oil having the following characteristics: $R_f$ = 0.14 [cyclohexane/ethylacetate (2:3 by volume)].

EXAMPLE 21 – N-[2-acetylthio-3-(4-fluorophenyl)-1-oxobutyl]tyrosine

Working as in Example 2, but starting with the product of Example 20, the titled compound is obtained in a 74% yield as an oily composition having the following characteristic: $R_f$ = 0.45 [methylene chloride/methanol/acetic acid (9:1:0.5 by volume)].

EXAMPLE 22 – N-[2-acetylthiomethyl-3-(3,4-difluorophenyl)-1-oxobutyl]alanine Benzyl Ester

Working as in Example 1, but starting with 2-acetylthiomethyl-3-(3,4-difluoro)phenyl butanoic acid and benzyl ester of alanine the title compound is formed as an oil having the following characteristic: $R_f$ = 0.2 [hexane/ethyl acetate (3:1 by volume)].

EXAMPLE 23 – N-[2-mercaptomethyl-3-(3,4-difluorophenyl)-1-oxobutyl]alanine

Working as in Example 2, but using the product of Example 22, the titled compound is obtained in a yield of 72% as a white solid having the following characteristics: m.p. 61 °C; $R_f$ = 0.17 [methylene

chloride/methanol (9:1 by volume)].

EXAMPLE 24 – N-[2-mercaptomethyl-3-(3,4-difluorophenyl)-1-oxobutyl]tyrosine Benzyl Ester

Working as in Example 1, but using the benzyl ester of tyrosine and 2-acetylthiomethyl-3-(3,4-difluoro)phenyl butanoic acid, the title compound is obtained in a yield of 78% as an oil having the following characteristic $R_f$ = 0.26 [cyclohexane/ethyl acetate (6:4 by volume)].

EXAMPLE 25 – N-[2-mercaptomethyl-3-(3,4-difluorophenyl)-1-oxobutyl]tyrosine

Working as in Example 2, but using the product of Example 24, the title compound is obtained in a yield of 80% as a pale yellow oil having the following characteristic: $R_f$ = 0.10 [methylene chloride/methanol (9:1 by volume)].

EXAMPLE 26 – Resolution of 2-Acetylthiomethyl-3-phenylbutanoic (ATBA) Acid to Four Diastereomers (I-IV)

(a) Resolution Using (R)-(+)-1-(1-Naphthyl)ethylamine to Yield ATBA-I

2-Acetylthiomethyl-3-phenylbutanoic acid (5 g) is dissolved in ethanol (50 mL) and (R)-(+)-1-(1-naphthyl)ethylainine (3.4 g) is added to the ethanol solution. Ethyl ether (50 mL) is then added. The mixture is cooled in an ice bath and after scratching the reaction vessel a white precipitate is formed. The solution is stored at about 0° C for about 18 hours. The precipitate is filtered and washed with ethyl ether.

1.47 g of a white precipitate is obtained. The precipitate is recrystallized from absolute ethanol to yield 0.42 g of off-white crystals of a diastereomer, (R)-(+)-1-(1-naphthyl)ethylamonium 2-acetylthiomethyl-3-phenylbutanoate salt (ATBA-I salt).

The free acid ATBA-I is regenerated by contacting the ATBA-I salt (50 mg) aqueous acid (10% hydrochloric) end extracting with methylene chloride. The methylene chloride is evaporated and 32.1 mg of ATBA-I is obtained as an oil having the following characteristic: $[\alpha]_D^{RT}$ = +78.25 [28.5 mg in methanol (1 mL)]

(b) Resolution Using (S)-(-)-1-(1-Naphthyl)ethylamine to Yield ATBA-II

The procedure is as in Example 26(a) above, except that (S)-(-)-1-(1-naphthyl)ethylamine is used as the amine. 1.2 g of a white precipitate (ATBA-II salt) is obtained and following recrystallization 0.31 g is obtained.

The free acid (ATBA-II) is regenerated from the salt (50 mg) in a yield of 28.7 mg as an oil having the following characteristic $[\alpha]_D^{RT}$ = -79.38 [29.1 mg in methanol (1 mL)].

(c) Resolution Using (S)-Methylbenzylamine to Yield ATBA-III

2-Acetylthiomethyl-3-methylbutanoic acid (5 g) is dissolved in ethanol (35 mL) and a solution of (S)-methylbenzylamine (2.4 g) in methanol (10 mL) is added. The mixture is diluted to 200 mL with ethyl ether. A precipitate forms and the solution is cooled at about 0° C for about 18 hours.

The colorless crystalline product is filtered, washed with ethyl ether and dried to yield 2 g of ATBA-III salt. The product is recrystallized twice from a mixture of ethyl acetate/ethanol (2:1) (25 mL) to yield 1 g of ATBA-III salt.

The free acid ATBA-III is regenerated by contacting the ATBA III salt with aqueous acid (10% hydrochloric acid) and extracting with methylene chloride. The methylene is evaporated and 0.67 g of ATBA-III is obtained as a colorless crystalline solid.

(d) Resolution Using (R)-Methylbenzylamine to Yield ATBA-IV

The procedure in Example 26(c) above is used except that (R)-methylbenzylamine is used to obtain 1.1 g of ATBA-IV is obtained as a colorless crystalline solid..

The acid ATBA-I is regenerated by contacting the ATBA-I salt (50 mg) aqueous acid (10% hydrochloric) and extracting with methylene chloride. The methylene chloride is evaporated and 32.1 mg of ATBA-I is obtained as an oil having the following characteristic: $[\alpha]_D^{RT}$ = +78.25 [28.5 mg in methanol (1

mL)]

(b) Resolution Using (S)-(-)-1-(1-Naphthyl)ethylamine to Yield ATBA-II

The procedure is as in Example 26(a) above, except that (S)-(-)-1-(1-naphthyl)ethylamine is used as the amine.

1.2 g of a white precipitate (ATBA-II salt) is obtained and following recrystallization 0.31 g is obtained.

The regenerated of the free acid (ATBA-II) from the salt (50 mg) yielded 28.7 mg of ATBA-II as an oil having the following characteristic $[\alpha]_D^{RT}$ = -79.38 [29.1 mg in methanol (1 mL)].

(c) Resolution Using (S)-Methylbenzylamine to Yield ATBA-III

2-Acetylthiomethyl-3-methylbutanoic acid (5 g) is dissolved in ethanol (35 mL) and a solution of (S)-methylbenzylamine (2.4 g) in methanol (10 mL) is added. The mixture is diluted to 200 mL with ethyl ether. A precipitate forms and the solution is cooled at about 0°C for about 18 hours.

The colorless crystalline product is filtered, washed with ethyl ether and dried to yield 2 g of ATBA-III salt. The product is recrystallized twice from a mixture of ethyl acetate/ethanol (2:1) (25 mL) to yield 1 g of ATBA-III salt.

The acid ATBA-III is regenerated by contacting the ATBA-III salt with aqueous acid (10% hydrochloric acid) and extracting with methylene chloride. The methylene is evaporated and 0.67g of ATBA-III is obtained as a colorless crystalline solid.

(d) Resolution Using (R)-Methylbenzylamine to Yield ATBA-IV

The procedure in Example 26(c) above is used except that (R)-methylbenzylamine is used to obtain 1.1 g of ATBA-IV as a colorless crystalline solid.

EXAMPLE 27 - Preparation at Four Diastereomers of N-(2-Acetylthiomethyl-1-oxo-3-phenylbutyl)-tyrosine Benzyl Ester (ATBAT-(I-IV)) by Synthetic Coupling of Resolved Reactants

(a) N-(2-Acetylthiomethyl-1-oxo-3-phenylbutyl)tyrosine Benzyl Ester (ATBAT-IV)

The product (ATBA-IV) (0.2 g) of Example 26 (b) is dissolved in methylene chloride (5 mL). (S)-Tyrosine benzyl ester p-tosylate (0.35 g), 1-(3-dimethylaminopropyl)-3-ethylcarbodiimide (0.15 g), hydroxybenzotriazole (0.11 g) and N-methylmorpholine (0.19 g) are added to the solution and the mixture is stirred at 20°C for 18 hours.

The solvent is removed *in vacuo*, the residue is contacted with aqueous acid (10% hydrochloric acid) and extracted with ethylacetate. The organic phase is separated and washed consecutively with water (20 mL) saturated sodium bicarbonate solution (20 mL) and water (20 mL). The organic phase is dried and evaporated to yield a light yellow gum. The gum is chromatographed on silica gel using a mixture of hexane/ethyl acetate (1:1) to yield a colorless gum (ATBAT-IV) (0.26 g; 65%) having the following characteristic: $[\alpha]_D^{25}$ = +98.90.

(b) N-(2-Acetylthiomethyl-1-oxo-3-phenylbutyl)tyrosine Benzyl Ester (ATBAT-III)

Using the procedure of Example 27(a), except starting with the product (ATBA-III) of Example 26(a), the product (ATBAT-III) is obtained after crystallization having the characteristics: m.p. 151-153°C; $[\alpha]_D^{25}$ = -89.7°C.

(c) N-(2-Acetylthiomethyl-1-oxo-3-phenylbutyl)tyrosine Benzyl Ester (ATBAT-II)

Using the procedure of Example 27(a), except starting with the product (ATBA-II) of Example 26(b), the product (ATBAT-II) is obtained.

(d) N-(2-Acetylthiomethyl-1-oxo-3-phenylbutyl)tyrosine Benzyl Ester (ATBAT-I)

Using the procedure of Example 27(a), except starting with the product (ATBA-I) of Example 26(a), the product (ATBAT-I) is obtained after crystallization having the characteristics: m.p. 135-137°C.

EXAMPLE 28 – Preparation of Diastereomers of N–(2–acetylthiomethyl–1–oxo–3–phenylbutyl)–tyrosine Benzyl Ester (ATBAT–(1–4)) by Column Chromatography

The diastereomers of N–(2–acetylthiomethyl–1–oxo–3–phenylbutyl)tyrosinebenzyl ester are also obtained by column chromatography using Hewlett–Packard ODS Hypersil column (5 μ, 200 x 4.6 mm i.d.). The product of Example 1 is dissolved in methanol and placed on the column. A mobile phase of methanol/water (62:38 by volume) with 0.2% trifluoroacetic acid is employed in effecting the separation. The flow rate is 1.0 mL/min. The product is detected at UV of 215 nm. The retention times of the four diastereomers are 22.7, 24.75, 28.5 and 30 minutes respectively.

EXAMPLE 29 – Two Diastereomers of N–(2–mercaptothiomethyl–1–oxo–3–phenylbutyl)tyrosine (MTBT–(I–II))

(a) N–(2–mercaptothiomethyl–1–oxo–3–phenylbutyl)tyrosine (MTBT–(I–II))

Lithium hydroxide hydrate (18 mg) is dissolved in solvent composing tetrahydrofuran/methanol/water (1:1:1 by volume). The solvent is purged with nitrogen gas. The product (ATBAT–IV) (21 mg) of Example 27(a)is added to the solution and stirred for 75 minutes at 20° C. The solution is then diluted with water (5 mL), washed twice with chloroform (3 mL), acidified with 10% hydrochloric acid (2 mL) and extracted five times with chloroform (4 mL). The combined organic phases are dried over magnesium sulphate and evaporated to leave 16 mg of a colorless gum (MTBT–I) having > 95% purity.

(b) N–(2–mercaptothiomethyl–1–oxo–3–phenylbutyl)tyrosine (MTBT–(I–II))

Using the procedure of Example 28(a), except starting with the product (ATBAT–II) of Example 27(b), 9.7 mg the product (MTBT–II) is obtained after crystallization having > 98% purity.

When administered to mammals, the products of formula (I) are very potent antihypertensives owing to their dual inhibitory action on neutral endopeptidase and peptidyldipeptidase A.

Peptidyldipeptidase A inhibitors are known products for treating certain forms of hypertension: they are capable of blocking the rise in blood pressure caused by an increase in vascular resistance and blood volume due to the formation of angiotensin II from angiotensin I.

Another important peptide involved in the regulation of arterial blood pressure is atrial natriuretic peptide, which is released by the heart, which is endowed with a vasodilator property and is capable of controlling diuresis and natriuresis. Atrial natriuretic peptide is degraded by neutral endopeptidase in the peripheral tissues. Neutral endopeptidase inhibitors such as thiorphan induce significant diuresis and natriuresis in man without giving rise to an increase in the renin and aldosterone levels, which is normally observed with diuretics generally used in combination with ACE inhibitors. However, the effects on blood pressure of neutral endopeptidase inhibitors used alone are small.

Mixed inhibitors of neutral endopeptidase and of peptidyldipeptidase A can relieve human hypertension of diverse origins and may be used without coadministration of diuretics.

Thus, the new products according to the present invention are very effective in the treatment of congestive heart failure and of various types of hypertension, especially hypertension linked to an increase in blood volume.

The products according to the invention possess the dual property of inhibiting both neutral endopeptidase and peptidyldipeptidase A. As a result, the hypotensive effect obtained in these mixed inhibitors is greater than that which is obtained with neutral endopeptidase and peptidyldipeptidase A inhibitors used alone or mixed.

The inhibitory powers of the products according to the invention are measured using $[^3H]$–D–$Ala^2$–Leu–enkephalin for neutral endopeptidase as described previously by Llorens et al., Biochem. Biophys. Res. Common., 96, 1710 (1980), and Z–Phe–His–Leu as substrate in the case of peptidyldipeptidase A according to the method described in Biochem. Biophys. Acta., 206, 136–142 (1970).

The antihypertensive effect of the products is determined in rats suffering from hypertension induced by DOCA/salt administration, and in spontaneously hypertensive male rats (SHR) according to Trapani et al., J. Cardiovasc. Pharmacol., 14, 419–424 (1989).

The results are coilated in Table I, which also gives the results with N–(2–mercaptomethyl–3–phenyl–1–oxopropyl)glycine (thiorphan) which, though of very closely related structure, manifests activity only against neutral endopeptidase (NEP).

TABLE I

| Inhibitory effect on neutral endopeptidase (NEP) and peptidyldipeptidase A (ACE) | | |
|---|---|---|
| Example | $IC_{50}$(nM) NEP | $IC_{50}$(nM) ACE |
| 2 | 2.5 x $10^{-9}$ | 1.8 x $10^{-9}$ |
| 4 | 1.3 x $10^{-9}$ | 2.5 x $10^{-8}$ |
| 6 | 4 x $10^{-9}$ | 7.1 x $10^{-8}$ |
| 8 | 2 x $10^{-9}$ | 2.8 x $10^{-9}$ |
| 10 | 3.1 x $10^{-9}$ | 5.8 x $10^{-9}$ |
| 12 | 1.3 x $10^{-9}$ | 7.9 x $10^{-9}$ |
| 14 | 3.5 x $10^{-9}$ | 6.2 x $10^{-9}$ |
| 16 | 1.8 x $10^{-9}$ | 3.5 x $10^{-9}$ |
| 19 | 1.9 x $10^{-9}$ | 8.5 x $10^{-9}$ |
| 21 | 4.8 x $10^{-9}$ | 5.9 x $10^{-9}$ |
| 23 | 3 x $10^{-9}$ | 5 x $10^{-9}$ |
| 25 | 6.3 x $10^{-9}$ | 3.5 x $10^{-9}$ |
| 29(a) | 1 x $10^{-9}$ | 3 x $10^{-9}$ |
| 29(b) | 3.1 x $10^{-9}$ | 52 x $10^{-9}$ |
| Thiorphan | 2.0 x $10^{-9}$ | 140 x $10^{-9}$ |

The products according to the invention may generally be administered orally or parenterally for the treatment of patients suffering from hypertension.

The products according to the invention, in base or salt form, may be presented in forms permitting administration by the most suitable route and the invention also relates to pharmaceutical compositions containing at least one product according to the invention which are suitable for use in human or veterinary medicine. These compositions may be prepared according to the customary methods, using one or more pharmaceutically acceptable adjuvants or excipients. The adjuvants comprise, inter alia, diluents, sterile aqueous media and the various non-toxic organic solvents. The compositions may be presented in the form of tablets, pills, granules, powders, aqueous solutions or suspensions, injectable solutions, elixirs or syrups, and can contain one or more agents chosen from the group comprising sweeteners, flavorings, colorings, or stabilizers in order to obtain pharmaceutically acceptable preparations.

The choice of vehicle and the content of active substance in the vehicle are generally determined in accordance with the solubility and chemical properties of the product, the particular mode of administration and the provisions to be observed in pharmaceutical practice. For example, excipients such as lactose, sodium citrate, calcium carbonate, dicalcium phosphate and disintegrating agents such as starch, alginic acids and certain complex silicates combined with lubricants such as magnesium stearate, sodium lauryl sulphate and talc may be used for preparing tablets. To prepare a capsule, it is advantageous to use lactose and high molecular weight polyethylene glycols. When aqueous suspensions are used they can contain emulsifying agents or agents which facilitate suspension. Diluents such as ethanol, propylene glycol, glycerol and chloroform or mixtures thereof may also be used.

For parenteral administration, suspensions or solutions of the products according to the invention in sesame oil, groundnut oil or olive oil or aqueous solutions of propylene glycol, as well as sterile aqueous solutions of the pharmaceutically acceptable salts, are used. The solutions of the salts of the products according to the invention are especially useful for administration by intramuscular or subcutaneous injection. The aqueous solutions, also comprising solutions of the salts in pure distilled water, may be used for intravenous administration with the proviso that their pH is suitably adjusted, that they are judiciously buffered and rendered isotonic with a sufficient quantity of glucose or sodium chloride and that they are sterilized by heating or microfiltration.

The doses used in the methods according to the invention are those which lead to a maximal therapeutic effect until an improvement is obtained, in general, the doses used are those which are therapeutically effective for lowering blood pressure during the treatment of hypertension. In general, the doses administered orally are between 0.1 and 100 mg/kg, and preferably between 1 and 10 mg/kg, and those administered intravenously are between 0.01 and 10 mg/kg, and preferably between 0.1 and 5 mg/kg, on the understanding that, in each particular case, the doses will be determined in accordance with the factors distinctive to the subject to be treated, such as age, weight, general state of health and other characteristics which can influence the efficacy of the medicinal product.

The products according to the invention may be administered as frequently as necessary in order to obtain the desired therapeutic effect. Some patients may respond rapidly to a higher or lower dose and may find much weaker maintenance doses adequate. For other patients, it may be necessary to have long-term treatments at the rate of 1 to 4 doses per day. In accordance with the physiological requirements of each particular patient. Generally, the active product may be administered orally 1 to 4 times per day. It goes without saying that, for other patients, it will be necessary to prescribe not more than one or two doses per day.

The products according to the invention may be used in injectable form in emergency cases of acute hypertension. Such a treatment may be followed by an intravenous perfusion of the active product so as to obtain and maintain the desired therapeutic effect.

The present invention may be embodied in other specific forms without departing from the spirit or essential attributes thereof and, accordingly, reference should be made to the appended claims, rather than the specification, as indicating the scope of the invention.

## Claims

**1.** A compound of formula (I)

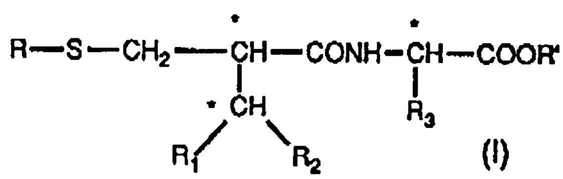

(I)

wherein

R represents a hydrogen atom or an acyl, aroyl or cycloalkylcarbonyl radical or a residue of formula

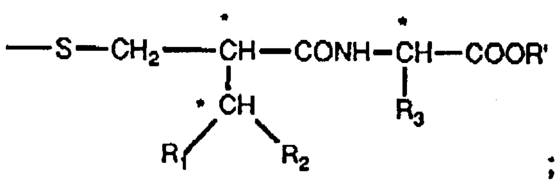

;

$R_1$ represents an alkyl radical;

$R_2$ represents an aryl or heteroaryl radical, or $R_1$ taken together with a carbon atom in an ortho position of the $R_2$ aryl or heteroaryl radical with respect to a carbon of the $R_2$ aryl or heteroaryl radical linked to the propanoyl moiety forms an alkylene chain;

$R_3$ represents a hydrogen atom or an alkyl, aryl, alkoxy or aryloxy radical; and

R' represents a hydrogen atom or an alkyl, aralkyl, acyl or aroyl radical;

and the pharmaceutically acceptable salts thereof.

**2.** The compound of claim 1 wherein

R represents a hydrogen atom or an acyl radical, a benzoyl radical in which the phenyl ring is optionally substituted with a halo atom, a hydroxyl radical, an alkoxy radical, an amino radical, a dialkylamino radical or a trifluoromethyl radical, an adamantoyl, palmitoyl or pamoyl radical or a residue of formula (Ia) above;

$R_1$ represents a methyl or trifluoromethyl radical;

$R_2$ represents

- either a phenyl, 2-, 3- or 4-pyridyl, N-methyl-2- or -3-pyrrolyl, 2-or 3-fury or 2- or 3-thienyl radical optionally substituted, at a position other than the ortho position with respect to the carbon linked to the propanoyl chain, with one or more identical or different atoms or radicals chosen from a halo atom and hydroxyl radical, an acyloxy radical, an alkoxy radical, a phenoxy radical, a phenylthio radical and an amino radical, a dialkylamino radical, methylenedioxy radical

or ethylenedioxy radicals,

- or a phenyl, 2 - ,3 - or 4 - pyridyl, N - methyl - 2 - or - 3 - pyrrolyl, 2 - or 3 - furyl or 2 - or 3 - thienyl radical in which the carbon atom at the ortho position with respect to the carbon atom linked to the propanoyl chain forms with R1 an alkylene radical optionally substituted with a methyl radical or a radical - CH2 - Y or - Y - CH2 - in which Y represents an oxygen or sulphur atom or a nitrogen atom substituted with a methyl radical;

$R_3$ represents a hydrogen atom or a trifluoromethyl radical or

- alkyl radical, optionally substituted with a phenyl radical, a hydroxyl radical, an alkoxy radical, a phenoxy radical, an alkylthio radical, a phenylthio radical, a benzyloxy radical or benzylthio radical,
- an alkoxy radical, or
- a phenoxy radical, or
- an aryl radical,

provided that the phenyl radical or aryl radical and the phenyl portion of the phenoxy radical, phenylthio radical, benzyloxy radical or benzylthio radical are optionally substituted with one or more identical or different atoms or radicals chosen from a halo atom, a hydroxyl radical, an alkoxy radical, an amino radical, a dialkylamino radical, methylenedioxy radical or ethylenedioxy radical; and

R' represents a hydrogen atom or a methyl radical, ethyl radical, benzyl radical, cyclohexylmethyl radical, palmitoyl radical or pamoyl radical.

**3.** The compound of claim 1 selected from the group consisting of N - (2 - acetylthiomethyl - 1 - oxo - 3 - phenylbutyl)tyrosine, of N - (2 - acetylthiomethyl - 1 - oxo - 3 - phenylbutyl)glycine, of N - (2 - acetylthiomethyl - 1 - oxo - 3 - phenylbutyl) - O - benzylserine, of N - (2 - acetylthiomethyl - 1 - oxo - 3 - phenylbutyl)alanine, of N - (2 - acetylthiomethyl - 1 - oxo - 3 - phenylbutyl)norleucine, of N - [3 - acetylthio - 2 - (1 - indanyl) - 1 - oxopropyl]alanine, of N - [2 - acetylthiomethyl - 3 - (4 - hydroxyphenyl) - 1 - oxobutyl]alanine and of N - [2 - acetylthiomethyl - 3 - (4 - hydroxyphenyl) - 1 - oxobutyl] - tyrosine.

**4.** The compound of claim 1 selected from the group consisting of N - (2 - Mercaptomethyl - 1 - oxo - 3 - phenylbutyl)tyrosine, N - (2 - mercaptomethyl - 1 - oxo - 3 - phenylbutyl)glycine, N - (2 - mercaptomethyl - 1 - oxo - 3 - phenylbutyl) - O - benzylserine, N - (2 - mercaptomethyl - 1 - oxo - 3 - phenylbutyl)alanine, N - (2 - mercaptomethyl - 1 - oxo - 3 - phenylbutyl)norleucine, N - [3 - mercapto - 2 - (1 - indanyl) - 1 - oxopropyl]alanine, N - [2 - mercaptomethyl - 3 - (4 - hydroxyphenyl) - 1 - oxobutyl]alanine and N - [2 - (1 - - mercaptomethyl - 3 - (4 - hydroxyphenyl) - 1 - oxobutyl]tyrosine.

**5.** The compound of claim 1 having a stereochemical designation selected from the group consisting of R,R,S, S,R,S, S,S,S and R,S,S.

**6.** The compound of claim 5 having a stereochemical designation selected from the group consisting of S,S,S and R,S,S.

**7.** Process for preparing the compound of claim 1 comprising acylating an amino acid of formula

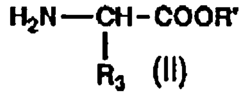

(II)

with an acid of general formula (III)

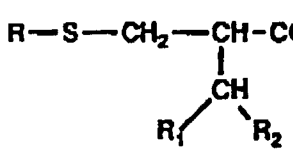

(III)

followed by replacement, where appropriate, of the radicals R and R' by hydrogen atoms, and the

product obtained is then isolated, where appropriate, in salt form.

**8.** A pharmaceutical composition comprising a compound of claim 1 and a pharmaceutically acceptable carrier.

**9.** The method of treating hypertension in a mammal comprising administering an effective antihypertensive amount of a compound of claim 1.

European Patent Office

# PARTIAL EUROPEAN SEARCH REPORT

which under Rule 45 of the European Patent Convention shall be considered, for the purposes of subsequent proceedings, as the European search report

Application Number

EP 92 11 7955

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| A | FR-A-2 616 070 (E.R. SQUIBB & SONS, INC.)<br>* page 30, line 10 - page 31, line 19; claims 1-4,6,7 *<br>--- | 1,8 | C 07 C 323/51<br>C 07 C 327/32<br>A 61 K 31/195<br>A 61 K 31/265 |
| A | CHEMICAL AND PHARMACEUTICAL BULLETIN. vol. 35, no. 6, 1987, TOKYO JP pages 2388 - 2393 TAKETOSHI KOMORI ET AL. 'sulfur containing acylamino acids. II...'<br>* the whole document *<br>--- | 1,8 | |
| A<br>D | EP-A-0 364 767 (SCHERING CORPORATION)<br>* abstract *<br>& US-A-4 879 309<br>--- | 1,8 | |
| A,D | EP-A-0 136 883 (E.R. SQUIBB & SONS, INC.)<br>* abstract; claims 1,19 *<br>--- -/- | 1,8 | |
| | | | TECHNICAL FIELDS SEARCHED (Int. Cl.5) |
| | | | C 07 C<br>A 61 K |

## INCOMPLETE SEARCH

The Search Division considers that the present European patent application does not comply with the provisions of the European Patent Convention to such an extent that it is not possible to carry out a meaningful search into the state of the art on the basis of some of the claims

Claims searched completely :

Claims searched incompletely :

Claims not searched :

Reason for the limitation of the search:

Remark: Although claim 9 is directed to a method of treatment of (diagnostic method practised on) the human/animal body (Art. 52(4) EPC) the search has been carried out and based on the alleged effects of the compound/ composition

| Place of search | Date of completion of the search | Examiner |
|---|---|---|
| BERLIN | 02-12-1992 | RUFET J M A |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0407)

European Patent Office

# PARTIAL EUROPEAN SEARCH REPORT

Application Number

EP 92 11 7955

| DOCUMENTS CONSIDERED TO BE RELEVANT | | | CLASSIFICATION OF THE APPLICATION (Int. Cl.5) |
|---|---|---|---|
| Category | Citation of document with indication, where appropriate, of relevant passages | Relevant to claim | |
| A,D | NATURE. vol. 288, 20 November 1980, LONDON GB pages 286 - 288 B.P. ROQUES ET AL. 'the enkephalinase inhibitor thiorphan shows antinociceptive activity in mice' * the whole document * ----- | 1,8 | |
| | | | TECHNICAL FIELDS SEARCHED (Int. Cl.5) |